# EUROPEAN PATENT SPECIFICATION

(11) **EP 3 705 123 B1**
(45) Date of publication and mention of the grant of the patent: **27.08.2025**
(21) Application number: 18872507.1
(22) Date of filing: 29.10.2018
(51) Int. Cl.: A61K 31/568, A61K 9/70, A61K 47/02, A61K 47/10, A61K 47/14, A61K 47/24, A61K 47/26, A61K 47/32, A61K 47/38, A61P 3/00, A61P 3/04, A61P 3/10, A61P 5/26, A61P 5/50, A61P 9/10, A61P 15/10, A61P 15/12, A61P 19/10, A61P 21/00, A61P 25/24, A61P 25/28

(54) **TRANSDERMALLY ADMINISTRABLE PREPARATION OF TESTOSTERONE**
TRANSDERMAL VERABREICHBARE ZUBEREITUNG VON TESTOSTERON
PRÉPARATION DE TESTOSTÉRONE POUVANT ÊTRE ADMINISTRÉE PAR VOIE TRANSDERMIQUE

(30) Priority: 30.10.2017 JP 2017209588
(43) Date of publication of application: 09.09.2020
(73) Proprietor: Teikoku Seiyaku Co., Ltd., Higashikagawa-shi, Kagawa 769-2695 (JP)
(72) Inventor: KAWAKAMI, Satoshi, Higashikagawa-shi Kagawa 769-2695 (JP); HORIKAWA, Yasushi, Higashikagawa-shi Kagawa 769-2695 (JP)
(74) Representative: Kraus & Lederer PartGmbB
(86) International application number: PCT/JP2018/040065
(87) International publication number: WO 2019/088010

(56) References cited:
- EP-A1- 2 491 931
- WO-A1-2012/101016
- WO-A1-2013/067346
- WO-A1-2016/042413
- CN-A- 103 301 093
- JP-A- 2008 512 425
- JP-A- 2011 026 227
- JP-A- 2011 026 227
- JP-A- H09 505 278
- US-A1- 2004 053 901
- US-A1- 2014 182 597
- US-B1- 7 045 145
- DOHI M ET AL: "Enhancing Effects of Myristyl Lactate and Lauryl Lactate on Percutaneous Absorption of Indomethacin in Rats", CHEM PHARM BULL, vol. 38, no. 10, 1 October 1990 (1990-10-01), pages 2877 - 2879, XP055614558, DOI: 10.1248/cpb.38.2877
- DOHI, M. ET AL.: "Enhancing Effects of Myristyl Lactate and Lauryl Lactate on Percutaneous Absorption of Indomethacin in Rats", CHEM. PHARM. BULL., vol. 38, no. 10, 1990, pages 2877 - 2879, XP055614558

## Description

### TECHNICAL FIELD

The present invention relates to transdermal formulations containing testosterone having excellent skin permeability of testosterone and excellent formulation properties.

### BACKGROUND ART

Testosterone is a male hormone (androgen) produced in a testicle. Androgen plays many important physiological roles, and affects muscles, bones, central nervous systems, prostate glands, bone marrows, sexual functions, and the like. Testosterone replacement therapies have been carried out by injections, gels, and reservoir type formulations in the treatment of hypogonadism caused by congenital or acquired testosterone deficiency, as well as disease(s) or symptom(s) caused by the reduction of testosterone concentration associated with aging such as male menopause, metabolic syndrome, diabetes, insulin resistance, obesity, arteriosclerosis, osteoporosis, muscle weakness, cognitive decline, memory disorder, and depression. However, injections require frequent hospital visits, and have difficulty in maintaining physiological blood concentration. Also, gel has problems such as difficulty in precise administration and secondary exposure of the drug administered to a skin to others.

On the other hands, patches, especially matrix-type transdermal formulations can stably administer a certain amount of drugs. Also, they can administer desired drugs and interrupt or discontinue the administration just by simple procedures of application and release, and thus have almost no risk of contaminating others. As testosterone-containing matrix-type transdermal formulations, patches using bases such as acrylic adhesives have been studied to date [Patent Document 1 and Patent Document 2]. However, pressure sensitive adhesives such as acrylic adhesives, rubber adhesives, and silicone adhesives have low drug releasability from the formulations and thus need to contain a large amount of solubilizer(s) of the drugs and absorption enhancer(s) of the drugs in the formulations in order to enhance the drug releasability. As a result, there is a problem of worsened formulation properties.

Also, in the technical field of patch, nonaqueous patches containing various drugs comprising water-soluble polymers such as polyacrylic acid as the main bases have been studied [Patent Document 3]. These patches using water-soluble polymers or the like as bases (hereinafter also referred to as "nonaqueous bases") can contain a relatively large amount of hydrophilic solvents such as polyhydric alcohol, and thus are believed to be suitable for containing drugs which are relatively highly soluble in hydrophilic solvents. However, many absorption enhancers are oily solvents in general, and thus when these nonaqueous bases are mixed with transdermal absorption enhancers, the solvents per se are separated from the formulations, and the oily solvents exude from the formulations, which causes a problem of impaired formulation properties such as decreased adhesive force. Also, when surfactants are contained in the formulations in order to suppress the exudation of the oily solvents from the formulations, the surfactants cause adverse effects such as cutaneous irritation. For example, a lactic acid ester is known to be an excellent absorption enhancer of testosterone [Patent Document 4]. However, when a lactic acid ester is contained in the formulations, the lactic acid ester exudes from the formulations during storage. Thus, there is a problem that when the formulations are used and release liners are peeled off, liquid ingredients are adhered to entire release liners, and formulation properties decrease.
Further related systems and techniques for transdermal delivery are disclosed in US 2004/0053901 A, WO 2016/042413 A1, EP 2 491 931 A1, Dohi et al. Chem. Pharm. Bull. 1990 38(10), 2877-2879, US 2014/0182597 A1, WO 2013/067346 A1, and US 7 045 145 B1.

### Citation List

### Patent Document

Patent Document 1: JP 2002-542277 A
Patent Document 2: JP 2004-517965 A
Patent Document 3: JP 2011-26227 A
Patent Document 4: JPH 09-505278 A

### Summary of Invention

### Technical Problem

The present invention has been made in view of the above problems in the conventional art, and an object thereof is to provide transdermal formulations having high transdermal absorbability of testosterone and excellent formulation properties.

### Solution to Problem

The present inventors have earnestly studied in order to solve the above problems. As a result, they have found that a transdermal formulation according to claim 1 in which the formulation properties is not impaired and transdermal absorbability of testosterone is excellent can be provided by containing testosterone in a pasty preparation base which contains polyacrylic acid or a salt thereof, a thickener, and a cross-linking agent as the main base, and further contains propylene glycol as a plasticizer and a lactic acid ester as an absorption enhancer, and finally completed the present invention.

Namely, the present invention relates to the followings.

### 1. Transdermal formulation

[1] A transdermal formulation comprising testosterone; polyacrylic acid or a salt thereof; a thickener; a cross-linking agent; a plasticizer comprising propylene glycol; and a lactic acid ester in a pasty preparation.
   [1-1] The transdermal formulation according to [1], wherein the polyacrylic acid or a salt thereof is one or more selected from polyacrylic acid, sodium polyacrylate, and partially neutralized polyacrylic acid.
   [1-2] The transdermal formulation according to [1] or [1-1], wherein the thickener is one or more selected from carboxymethylcellulose sodium, hydroxyethyl cellulose, hydroxypropyl cellulose, hydroxymethyl cellulose, polyvinyl alcohol, polyvinylpyrrolidone, starch, and gelatin, preferably one or more selected from carboxymethylcellulose sodium, hydroxyethyl cellulose, hydroxypropyl cellulose, and hydroxymethyl cellulose.
   [1-3] The transdermal formulation according to any one of [1] to [1-2], wherein the cross-linking agent is one or more selected from dihydroxyaluminum aminoacetate, magnesium aluminometasilicate, aluminum hydroxide, and synthetic hydrotalcite, preferably one or more selected from dihydroxyaluminum aminoacetate, magnesium aluminometasilicate, and synthetic hydrotalcite.
   [1-4] The transdermal formulation according to any one of [1] to [1-3], wherein the lactic acid ester is one or more selected from methyl lactate, ethyl lactate, propyl lactate, butyl lactate, pentyl lactate, hexyl lactate, heptyl lactate, octyl lactate, nonyl lactate, decyl lactate, undecyl lactate, lauryl lactate, tridecyl lactate, myristyl lactate, pentadecyl lactate, cetyl lactate, heptadecyl lactate, and octadecyl lactate, preferably one or more selected from lauryl lactate, myristyl lactate, and cetyl lactate.
[2] The transdermal formulation according to [1], wherein
   the polyacrylic acid or a salt thereof is one or more selected from polyacrylic acid, sodium polyacrylate, and partially neutralized polyacrylic acid;
   the thickener is one or more selected from carboxymethylcellulose sodium, hydroxyethyl cellulose, hydroxypropyl cellulose, hydroxymethyl cellulose, polyvinyl alcohol, polyvinylpyrrolidone, starch, and gelatin;
   the cross-linking agent is one or more selected from dihydroxyaluminum aminoacetate, magnesium
      aluminometasilicate, aluminum hydroxide, and synthetic hydrotalcite; and
   the lactic acid ester is one or more selected from methyl lactate, ethyl lactate, propyl lactate, butyl lactate, pentyl lactate, hexyl lactate, heptyl lactate, octyl lactate, nonyl lactate, decyl lactate, undecyl lactate, lauryl lactate, tridecyl lactate, myristyl lactate, pentadecyl lactate, cetyl lactate, heptadecyl lactate, and octadecyl lactate.
[3] The transdermal formulation according to [1] or [2], wherein
   the polyacrylic acid or a salt thereof is one or more selected from polyacrylic acid, sodium polyacrylate, and partially neutralized polyacrylic acid;
   the thickener is one or more selected from carboxymethylcellulose sodium, hydroxyethyl cellulose, hydroxypropyl cellulose, and hydroxymethyl cellulose;
   the cross-linking agent is one or more selected from dihydroxyaluminum aminoacetate, magnesium aluminometasilicate, and synthetic hydrotalcite; and
   the lactic acid ester is one or more selected from lauryl lactate, myristyl lactate, and cetyl lactate.
[4] The transdermal formulation according to any one of [1] to [3], wherein the plasticizer further comprises one or more selected from ethanol, glycerin, 1,3-butylene glycol, polypropylene glycol, D-sorbitol, and polyethylene glycol 400.
[5] The transdermal formulation according to any one of [1] to [4], wherein the plasticizer further comprises one or more selected from ethanol, glycerin, and D-sorbitol.
[6] The transdermal formulation according to any one of [1] to [5], wherein the plasticizer is a combination of propylene glycol and ethanol.
[7] The transdermal formulation according to any one of [1] to [3], wherein
   the polyacrylic acid or a salt thereof is polyacrylic acid;
   the thickener is hydroxyethyl cellulose;
   the cross-linking agent is magnesium aluminometasilicate;
   the plasticizer is propylene glycol; and
   the lactic acid ester is one or more selected from lauryl lactate, myristyl lactate, and cetyl lactate.
[8] The transdermal formulation according to any one of [1] to [6], wherein
   the polyacrylic acid or a salt thereof is polyacrylic acid;
   the thickener is hydroxyethyl cellulose;
   the cross-linking agent is magnesium aluminometasilicate;
   the plasticizer is a combination of propylene glycol and ethanol; and
   the lactic acid ester is one or more selected from lauryl lactate, myristyl lactate, and cetyl lactate.
[9] The transdermal formulation according to any one of [1] to [8], wherein the amount of the testosterone is 0.01 to 10% by weight, the amount of the polyacrylic acid or a salt thereof is 1 to 20% by weight, the amount of the thickener is 0.5 to 10% by weight, the amount of the cross-linking agent is 0.02 to 5% by weight, the amount of the plasticizer is 45 to 90% by weight, and the amount of the lactic acid ester is 0.1 to 10% by weight, relative to the pasty preparation weight.
[10] The transdermal formulation according to any one of [1] to [9], wherein the amount of the testosterone is 0.2 to 4% by weight, the amount of the polyacrylic acid or a salt thereof is 3 to 15% by weight, the amount of the thickener is 1 to 5% by weight, the amount of the cross-linking agent is 0.05 to 4% by weight, the amount of the plasticizer is 67 to 90% by weight, and the amount of the lactic acid ester is 0.5 to 5% by weight, relative to the pasty preparation weight.
[11] The transdermal formulation according to any one of [1] to [10], wherein the amount of the testosterone is 0.5 to 2% by weight, the amount of the polyacrylic acid or a salt thereof is 5 to 10% by weight, the amount of the thickener is 1 to 3% by weight, the amount of the cross-linking agent is 1 to 4% by weight, the amount of the plasticizer is 77 to 90% by weight, and the amount of the lactic acid ester is 1 to 4% by weight, relative to the pasty preparation weight.
   The present invention also relates to the followings.
[12] The transdermal formulation according to any one of [1] to [11], wherein the pasty preparation further comprises one or more ingredient(s) selected from a surfactant; an absorption enhancer other than a lactic acid ester; a preservative; an antioxidant; a refreshing agent; a silicon compound; an inorganic filler; a flavoring agent; and a colorant.
[13] The transdermal formulation according to any one of [1] to [11], wherein the pasty preparation consists of testosterone; polyacrylic acid or a salt thereof; a thickener; a cross-linking agent; a plasticizer comprising propylene glycol; and a lactic acid ester.
[14] The transdermal formulation according to any one of [1] to [13], wherein the pasty preparation does not comprise a pressure sensitive adhesive.
   The present invention also relates to the followings. 2. Use of transdermal formulation
[15] The transdermal formulation according to any one of [1] to [14] for use in the prevention or treatment of disease(s) or symptom(s) caused by the reduction of testosterone concentration.
[16] The transdermal formulation for use according to [15], wherein the disease(s) or symptom(s) caused by the reduction of testosterone concentration is/are one or more disease(s) or symptom(s) selected from hypogonadism, male menopause, metabolic syndrome, diabetes, insulin resistance, obesity, arteriosclerosis, osteoporosis, muscle weakness, cognitive decline, memory disorder, and depression.
[17] Use of the transdermal formulation according to any one of [1] to [14] in the manufacture of a medicament for the prevention or treatment of disease(s) or symptom(s) caused by the reduction of testosterone concentration.
[18] The use according to [17], wherein the disease(s) or symptom(s) caused by the reduction of testosterone concentration is/are one or more disease(s) or symptom(s) selected from hypogonadism, male menopause, metabolic syndrome, diabetes, insulin resistance, obesity, arteriosclerosis, osteoporosis, muscle weakness, cognitive decline, memory disorder, and depression.
[19] Use of the transdermal formulation in a method for preventing or treating disease(s) or symptom(s) caused by the reduction of testosterone concentration, the use comprising administering the transdermal formulation according to any one of [1] to [14].
[20] The use according to [19], wherein the disease(s) or symptom(s) caused by the reduction of testosterone concentration is/are one or more disease(s) or symptom(s) selected from hypogonadism, male menopause, metabolic syndrome, diabetes, insulin resistance, obesity, arteriosclerosis, osteoporosis, muscle weakness, cognitive decline, memory disorder, and depression.

### EFFECT OF INVENTION

According to the transdermal formulation of claim 1 comprising testosterone, polyacrylic acid or a salt thereof, a thickener, a cross-linking agent, a plasticizer comprising propylene glycol, and a lactic acid ester of the present invention, a testosterone-containing transdermal formulation having excellent skin permeability of testosterone and excellent formulation properties can be provided. The transdermal formulation of the present invention can efficiently prevent or treat disease(s) or symptom(s) caused by the reduction of testosterone concentration.

### Description of Embodiments

In the present description, "contain" or "comprise" may be used interchangeably with "compound". Also, in the present description, "contained amount" may be used interchangeably with "amount".

The transdermal formulation of the present invention comprises testosterone, polyacrylic acid or a salt thereof, a thickener, a cross-linking agent, a plasticizer comprising propylene glycol, and a lactic acid ester in a pasty preparation. The pasty preparation is usually a pasty composition which contains a pasty preparation base and testosterone as a drug. In the transdermal formulation of the present invention, a mixture of polyacrylic acid or a salt thereof, a thickener, and a cross-linking agent is referred to as the "main base", and a mixture further comprising a plasticizer comprising propylene glycol and a lactic acid ester as an absorption enhancer is referred to as "pasty preparation base".

The amount of the testosterone in the transdermal formulation of the present invention is not specifically limited as long as it is a concentration to achieve a therapeutic effect, but is usually within a range of 0.01 to 10% by weight, preferably 0.2 to 4% by weight, more preferably 0.5 to 2% by weight, relative to the pasty preparation weight. When the amount of the testosterone is less than 0.01% by weight, sufficient transdermal absorbability of the drug cannot be achieved. Meanwhile, when the amount is more than 10% by weight, undissolved drug is crystallized in the formulation, which causes decreased drug releasability and decreased formulation properties.

The polyacrylic acid or a salt thereof in the transdermal formulation of the present invention forms a cross-linked product by a cross-linking agent to enhance the adhesive force of the pasty preparation. According to the invention, the polyacrylic acid or a salt thereof include polyacrylic acid, sodium polyacrylate, and partially neutralized polyacrylic acid, and one of them or a combination of two or more of them may be used. In the present invention, polyacrylic acid is preferable.

The amount of the polyacrylic acid or a salt thereof in the transdermal formulation of the present invention is usually within a range of 1 to 20% by weight, preferably 3 to 15% by weight, more preferably 5 to 10% by weight, relative to the pasty preparation weight. The amount of less than 1% by weight is not preferable, because a sufficient three-dimensional network is not formed, and the resulting gel becomes soft. Also, the amount of more than 20% by weight is not preferable, because the pasty preparation layer becomes too hard, which causes decreased adhesive force.

The thickener in the transdermal formulation of the present invention has a function to adjust the shape retention property of the pasty preparation. According to the invention, the thickener includes cellulose derivatives selected from carboxymethylcellulose sodium, hydroxyethyl cellulose, hydroxypropyl cellulose, and hydroxymethyl cellulose; synthetic water-soluble polymers such as polyvinyl alcohol and polyvinylpyrrolidone; starch; and gelatin, and one of them or a combination of two or more of them may be used. In the present invention, one or more cellulose derivative(s) selected from carboxymethylcellulose sodium, hydroxyethyl cellulose, hydroxypropyl cellulose, and hydroxymethyl cellulose is/are preferable, and hydroxyethyl cellulose is more preferable.

The amount of the thickener in the transdermal formulation of the present invention is usually within a range of 0.5 to 10% by weight, preferably 1 to 5% by weight, more preferably 1 to 3% by weight, relative to the pasty preparation weight. The amount of the thickener of less than 0.5% by weight is not preferable, because the pasty preparation property decreases, and the shape retention property during the manufacture decreases. Also, the amount of more than 10% by weight is not preferable, because the viscosity of gel base increases, which caused decreased workability during the manufacture.

The cross-linking agent in the transdermal formulation of the present invention has a function to form a cross-linked product of the polyacrylic acid or a salt thereof to maintain the shape retention property of the pasty preparation, and selected from hardly soluble multivalent metal salts. Examples of the cross-linking agent include dihydroxyaluminum aminoacetate, magnesium aluminometasilicate, aluminum hydroxide, and synthetic hydrotalcite, and one of them or a combination of two or more of them may be used. In the present invention, one or more selected from dihydroxyaluminum aminoacetate, magnesium aluminometasilicate, and synthetic hydrotalcite is/are preferable, and magnesium aluminometasilicate is more preferable.

The amount of the cross-linking agent in the transdermal formulation of the present invention is usually within a range of 0.02 to 5% by weight, preferably 0.05 to 4% by weight, more preferably 1 to 4% by weight, relative to the pasty preparation weight. When the amount of the cross-linking agent is less than 0.02% by weight, a cross-linked product is not sufficiently formed, and the shape retention property of the pasty preparation worsens. Meanwhile, when the amount of the cross-linking agent is more than 5% by weight, too many cross-linked products are formed, and the adhesive property of the formulation worsens.

The lactic acid ester in the transdermal formulation of the present invention acts as a transdermal absorption enhancer to enhance the permeation of testosterone to a skin. Examples of the lactic acid ester include methyl lactate, ethyl lactate, propyl lactate, butyl lactate, pentyl lactate, hexyl lactate, heptyl lactate, octyl lactate, nonyl lactate, decyl lactate, undecyl lactate, lauryl lactate, tridecyl lactate, myristyl lactate, pentadecyl lactate, cetyl lactate, heptadecyl lactate, and octadecyl lactate, and one of them or a combination of two or more of them may be used. In the present invention, one or more selected from lauryl lactate, myristyl lactate, and cetyl lactate is/are preferable, and lauryl lactate is more preferable.

The amount of the lactic acid ester in the transdermal formulation of the present invention is usually within a range of 0.1 to 10% by weight, preferably 0.5 to 5% by weight, more preferably 1 to 4% by weight, relative to the pasty preparation weight. The amount of the lactic acid ester of less than 0.1% by weight is not preferable, because the drug absorption decreases. The amount of more than 10% by weight is not preferable, because the formulation properties worsens.

The transdermal formulation of the present invention comprises propylene glycol as a plasticizer. Propylene glycol acts not only as a plasticizer, but also as a solubilizer of the base ingredients. Also, propylene glycol is highly compatible with a lactic acid ester as compared to other plasticizers, and excellent in the action to prevent the lactic acid ester from separating and exuding from the formulation.

In the present invention, propylene glycol is compounded in combination with other plasticizer(s), i.e. plasticizer(s) which include ethanol, glycerin, 1,3-butylene glycol, polypropylene glycol, D-sorbitol, and polyethylene glycol 400, and one of them or a combination of two or more of them may be used. One or more selected from ethanol, glycerin, and D-sorbitol is/are preferable, and ethanol is more preferable.

The amount of the plasticizer comprising propylene glycol in the transdermal formulation of the present invention is usually within a range of 45 to 90% by weight, preferably 67 to 90% by weight, more preferably 77 to 90% by weight, relative to the pasty preparation weight. The amount of the plasticizer less than 45% by weight is not preferable, because the shape retention property of the pasty preparation decreases, exudation of the lactic acid ester from the pasty preparation cannot be prevented, and the adhesive property worsens. Also, the amount of the plasticizer of more than 90% by weight is not preferable, because the adhesive property and the shape retention property of the pasty preparation decrease.

Further, the transdermal formulation of the present invention may comprise various ingredients generally contained in conventional external preparations and the like. Examples of such ingredients include surfactants such as polyoxyethylene sorbitan fatty acid ester; absorption enhancers other than a lactic acid ester such as isopropyl myristate; preservatives such as p-hydroxybenzoate ester; antioxidants such as dibutylhydroxytoluene; refreshing agents such as L-menthol; silicon compounds such as light anhydrous silicic acid; inorganic fillers such as zinc oxide; flavoring agents such as mentha oil; and colorants such as yellow ferric oxide, and they may be appropriately compounded at an appropriate amount.

In one embodiment, the pasty preparation of the transdermal formulation of the present invention may further comprise one or more ingredient(s) selected from a surfactant; an absorption enhancer other than a lactic acid ester; a preservative; an antioxidant; a refreshing agent; a silicon compound; an inorganic filler; a flavoring agent; and a colorant.

In another embodiment, the pasty preparation of the transdermal formulation of the present invention consists of testosterone; polyacrylic acid or a salt thereof; a thickener; a cross-linking agent; a plasticizer comprising propylene glycol; and a lactic acid ester.

The pasty preparation of the transdermal formulation of the present invention does not comprise a pressure sensitive adhesive. Examples of such pressure sensitive adhesive include acrylic adhesives (for example, acrylate copolymers such as Duro-Tak 87-4098), rubber adhesives (for example, styrene-isoprene-styrene block copolymers), and silicone adhesives (for example, BIO-PSA 7-4302, BIO-PSA 7-4101, BIO-PSA 7-4102, and BIO-PSA 7-4202 manufactured by Dow Corning Corp.).

The transdermal formulation of the present invention is useful in the prevention or treatment of disease(s) or symptom(s) caused by the reduction of testosterone concentration. Examples of such disease(s) or symptom(s) include one or more disease(s) or symptom(s) selected from hypogonadism, male menopause, metabolic syndrome, diabetes, insulin resistance, obesity, arteriosclerosis, osteoporosis, muscle weakness, cognitive decline, memory disorder, and depression. The transdermal formulation of the present invention is preferably used in the prevention or treatment of hypogonadism or male menopause.

In the transdermal formulation of the present invention, the pasty preparation is spread or applied between a backing and a release liner. The backing used in the transdermal formulation of the present invention is preferably a highly flexible and thin backing which follows body movements in view of suppressing the release of the formulation after application. Examples thereof include, but are not limited to, various non-woven fabrics, woven fabrics, films, and sheets, and specific examples thereof to be used include woven fabrics or non-woven fabrics made from a fiber such as rayon, polyester, polyolefin, and urethane, and polymer films, foam sheets, and laminate films thereof.

Examples of the release liner to cover the surface of the pasty preparation include polyethylene, polypropylene, polyester, and those obtained by subjecting these materials into mold release treatment with silicon.

Also, the transdermal formulation of the present invention has a high adhesive force, and basically does not need auxiliary means for adhesion such as a cover sheet. However, when it is applied to a human over a long period of time, a cover sheet may be auxiliarily used in order to maintain a good adhesive property to a skin. The cover sheet is placed at the backing side of the formulation, and examples of the material thereof include non-woven fabric, cloth, net, knit, gauze, and film. Among them, materials having a certain degree of air permeability are preferable so as not to cause damp or rash at the adhesion site. Specific examples of the material of the cover sheet include polyester (fiber), polyethylene (fiber), polypropylene (fiber), rayon, cupra, and hemp. Also, examples of the adhesive used in the adhesive layer of the cover sheet include acrylic adhesives such as acrylic acid alkyl ester, rubber adhesives such as styrene-isoprene-styrene block copolymers, and silicone adhesives.

The testosterone-containing transdermal formulation of the present invention may be prepared according to, for example, the following method. First, polyacrylic acid or a salt thereof and thickener(s) such as hydroxyethyl cellulose are dissolved into plasticizer(s) such as propylene glycol with heating. The resulting solution is cooled, and then mixed with stirring a plasticizer in which testosterone and a lactic acid ester are dissolved and a cross-linking agent is dispersed to prepare a pasty preparation solution (hereinafter referred to as "nonaqueous pasty preparation"). Said nonaqueous pasty preparation is applied on a backing, then covered by a release liner, and cut into a desired size to prepare a transdermal formulation of the present invention. In the transdermal formulation of the present invention, the applied amount of the pasty preparation is 50 to 1000 g/m².

Next, the present invention is more specifically illustrated by means of Examples, but the present invention is not limited to the following Examples. In the Examples, numerical values are expressed in "% by weight", unless otherwise specified.

### EXAMPLES

### Examples 1 to 3

Polyacrylic acid and hydroxyethyl cellulose were added to propylene glycol to be dissolved therein with heating. After cooled, to the resulting solution was added ethanol in which testosterone and a lactic acid ester were dissolved and magnesium aluminometasilicate was dispersed, and the resulting mixture was mixed with stirring to prepare a nonaqueous pasty preparation. Said nonaqueous pasty preparation was homogeneously applied on a backing at an applied amount of 700 g/m², then covered by a release liner, and cut into a desired size to prepare a transdermal formulation of each Example.

### Example 4 (Reference Example)

A transdermal formulation was prepared according to the method for producing Examples 1 to 3, except for the solvent in which testosterone and a lactic acid ester were dissolved and magnesium aluminometasilicate was dispersed was changed to propylene glycol.

The ingredients contained in the formulation of each Example (Ex.) is shown in Table 1.

**Table 1**

| Contained ingredient | Ex. 1 | Ex. 2 | Ex. 3 | Ex. 4* |
|---|---|---|---|---|
| Testosterone | 1 | 1 | 1 | 1 |
| Ethanol | 30 | 30 | 30 | - |
| Lauryl lactate | 2 | - | - | 2 |
| Myristyl lactate | - | 2 | - | - |
| Cetyl lactate | - | - | 2 | - |
| Propylene glycol | 55.7 | 55.7 | 55.7 | 85.7 |
| Magnesium aluminometasilicate | 3 | 3 | 3 | 3 |
| Hydroxyethyl cellulose | 1.5 | 1.5 | 1.5 | 1.5 |
| Polyacrylic acid | 6.8 | 6.8 | 6.8 | 6.8 |
| Total | 100 | 100 | 100 | 100 |
| Pasty preparation property | ○ | Δ | Δ | ○ |

| | | | | |
|---|---|---|---|---|
| *) Reference Example | | | | |

### Comparative Example 1

A transdermal formulation was prepared according to the same manner as the Example 1, except for not adding lauryl lactate.

### Comparative Examples 2 and 3

Each transdermal formulation was prepared according to the method in the Example 1, except for the solvent for dissolving polyacrylic acid and hydroxyethyl cellulose with heating was changed to glycerin or sorbitol.

The ingredients contained in each formulation of Comparative Examples (Comp.) 1 to 3 are shown in Table 2.

**Table 2**

| Contained ingredient | Comp. 1 | Comp. 2 | Comp. 3 |
|---|---|---|---|
| Testosterone | 1 | 1 | 1 |
| Ethanol | 30 | 30 | 30 |
| Lauryl lactate | - | 2 | 2 |
| Propylene glycol | 57.7 | - | - |
| Glycerin | - | 57.2 | - |
| Sorbitol | - | - | 56.0 |
| Magnesium aluminometasilicate | 3 | 2.5 | 3 |
| Hydroxyethyl cellulose | 1.5 | 1.6 | 1.6 |
| Polyacrylic acid | 6.8 | 5.7 | 6.4 |
| Total | 100 | 100 | 100 |
| Pasty preparation property | ○ | × | × |

### Comparative Examples 4 and 5

Testosterone was dissolved into dimethyl sulfoxide to prepare a drug solution. Said drug solution and an acrylic adhesive (Duro-Tak 87-4098) were homogeneously mixed with stirring to prepare an adhesive solution, the resulting adhesive solution was spread on a release liner, the solvent was removed by drying, an adhesive layer having a thickness of 100 µm was formed, and then a backing was applied to prepare a tape. The ingredients contained in each formulation of Comparative Examples (Comp.) 4 and 5 are shown in Table 3.

**Table 3**

| Contained ingredient | Comp. 4 | Comp. 5 |
|---|---|---|
| Testosterone | 6 | 9 |
| Dimethyl sulfoxide | 9.4 | 18.2 |
| Duro-Tak 87-4098 | 84.6 | 72.8 |
| Total | 100 | 100 |
| Pasty preparation property | ○ | ○ |

### [Test Examples]

### Test Example 1: Observation of formulation properties after preparation (Observation of the presence or absence of exudation of liquid ingredient(s) from pasty preparation surface)

A release liner of each Example and each Comparative Example was released after 3 days from the preparation, the amount of liquid ingredient(s) adhered to the film after release was visually observed, and the pasty preparation property was evaluated to evaluate the formulation properties. The results are shown in the above Table 1 to Table 3. The evaluation criteria of this test are as follows.

### [Evaluation criteria of properties]

○: No liquid ingredient was adhered to the release liner.
Δ: A very small amount of liquid ingredient(s) was adhered
to the release liner.
×: Liquid ingredient(s) was/were adhered to almost the entire surface of the release liner.

### Test Example 2: In vitro skin permeation test in hairless rat

In order to study the transdermal absorbability of testosterone in the transdermal formulation of the present invention, each formulation of Examples and Comparative Examples 1, 2, 4, and 5 was subjected to an in vitro skin permeation test in a hairless rat. An excised abdominal skin of a male hairless rat (HWY strain, 7 weeks old) was put in a Franz diffusion cell, and each test formulation cut into a round shape (ϕ 28 mm) was applied to the skin. The receptor side was filled with phosphate buffered saline in which 40% polyethylene glycol was dissolved, and hot water of 37°C was circulated in the water jacket. The receptor solution was sampled with time, and the amount of testosterone permeated the skin was measured by liquid chromatography. Using the result, a cumulative permeation amount after 24 hours from the start of the test was calculated. The results are shown in Table 4.

**Table 4**

| Example / Comparative Example | Cumulative permeation amount of drug after 24 hours (µg/cm²) |
|---|---|
| Example 1 | 139.6 |
| Example 2 | 158.9 |
| Example 3 | 181.5 |
| Example 4* | 109.5 |
| Comparative Example 1 | 7.7 |
| Comparative Example 2 | 39.0 |
| Comparative Example 4 | 13.5 |
| Comparative Example 5 | 11.5 |

| | |
|---|---|
| *)Reference Example | |

### [Discussion]

According to the each test results described above, the transdermal formulation of each Example was found to be a formulation having excellent transdermal absorbability of testosterone and excellent formulation properties. On the other hands, the Comparative Example 1 which did not contain a lactic acid ester and the Comparative Example 4 and the Comparative Example 5 which contained an acrylic adhesive as the main base were found to have significantly poor transdermal absorbability of testosterone as compared to formulations of Examples. Also, the formulations of the Comparative Example 2 and the Comparative Example 3 which did not contain propylene glycol showed exudation of liquid ingredient(s) from the pasty preparation surfaces and significantly decreased formulation properties.

### INDUSTRIAL APPLICABILITY

According to the present invention, a testosterone-containing transdermal formulation having excellent drug releasability and very good formulation properties can be provided. The transdermal formulation of the present invention is very useful in, for example, the prevention or treatment of disease(s) or symptom(s) caused by the reduction of testosterone concentration.

## Claims

1. A transdermal formulation comprising testosterone; polyacrylic acid or a salt thereof; a thickener; a cross-linking agent; a plasticizer comprising propylene glycol; and a lactic acid ester in a pasty preparation, wherein
the polyacrylic acid or a salt thereof is one or more selected from polyacrylic acid, sodium polyacrylate, and partially neutralized polyacrylic acid;
the thickener is one or more selected from carboxymethylcellulose sodium, hydroxyethyl cellulose, hydroxypropyl cellulose, hydroxymethyl cellulose, polyvinyl alcohol, polyvinylpyrrolidone, starch, and gelatin;
the cross-linking agent is one or more selected from dihydroxyaluminum aminoacetate, magnesium aluminometasilicate, aluminum hydroxide, and synthetic hydrotalcite;
the plasticizer comprises propylene glycol and further comprises one or more selected from ethanol, glycerin, 1,3-butylene glycol, polypropylene glycol, D-sorbitol, and polyethylene glycol 400; and
the lactic acid ester is one or more selected from methyl lactate, ethyl lactate, propyl lactate, butyl lactate, pentyl lactate, hexyl lactate, heptyl lactate, octyl lactate, nonyl lactate, decyl lactate, undecyl lactate, lauryl lactate, tridecyl lactate, myristyl lactate, pentadecyl lactate, cetyl lactate, heptadecyl lactate, and octadecyl lactate;
and the pasty preparation does not comprise a pressure sensitive adhesive.

2. The transdermal formulation according to claim 1, wherein
the thickener is one or more selected from carboxymethylcellulose sodium, hydroxyethyl cellulose, hydroxypropyl cellulose, and hydroxymethyl cellulose;
the cross-linking agent is one or more selected from dihydroxyaluminum aminoacetate, magnesium aluminometasilicate, and synthetic hydrotalcite; and
the lactic acid ester is one or more selected from lauryl lactate, myristyl lactate, and cetyl lactate.

3. The transdermal formulation according to any one of claims 1 to 2, wherein the plasticizer comprises propylene glycol and further comprises one or more selected from ethanol, glycerin, and D-sorbitol.

4. The transdermal formulation according to any one of claims 1 to 3, wherein the plasticizer is a combination of propylene glycol and ethanol.

5. The transdermal formulation according to any one of claims 1 to 4, wherein
the polyacrylic acid or a salt thereof is polyacrylic acid;
the thickener is hydroxyethyl cellulose;
the cross-linking agent is magnesium aluminometasilicate;
the plasticizer is a combination of propylene glycol and ethanol; and
the lactic acid ester is one or more selected from lauryl lactate, myristyl lactate, and cetyl lactate.

6. The transdermal formulation according to any one of claims 1 to 5, wherein the amount of the testosterone is 0.01 to 10% by weight, the amount of the polyacrylic acid or a salt thereof is 1 to 20% by weight, the amount of the thickener is 0.5 to 10% by weight, the amount of the cross-linking agent is 0.02 to 5% by weight, the amount of the plasticizer is 45 to 90% by weight, and the amount of the lactic acid ester is 0.1 to 10% by weight, relative to the pasty preparation weight.

7. The transdermal formulation according to any one of claims 1 to 6, wherein the amount of the testosterone is 0.2 to 4% by weight, the amount of the polyacrylic acid or a salt thereof is 3 to 15% by weight, the amount of the thickener is 1 to 5% by weight, the amount of the cross-linking agent is 0.05 to 4% by weight, the amount of the plasticizer is 67 to 90% by weight, and the amount of the lactic acid ester is 0.5 to 5% by weight, relative to the pasty preparation weight.

8. The transdermal formulation according to any one of claims 1 to 7, for use in the prevention or treatment of one or more disease(s) or symptom(s) selected from hypogonadism, male menopause, metabolic syndrome, diabetes, insulin resistance, obesity, arteriosclerosis, osteoporosis, muscle weakness, cognitive decline, memory disorder, and depression.

## Patentansprüche

1. Transdermale Formulierung, umfassend Testosteron;
Polyacrylsäure oder ein Salz davon; ein Verdickungsmittel; ein Vernetzungsmittel; einen Propylenglykol umfassenden Weichmacher; und einen Milchsäureester in einer pastösen Zubereitung, wobei
die Polyacrylsäure oder das Salz davon eine oder mehrere ist, ausgewählt aus Polyacrylsäure, Natriumpolyacrylat und teilweise neutralisierte Polyacrylsäure;
das Verdickungsmittel eines oder mehrere ist, ausgewählt aus Carboxymethylcellulose-Natrium, Hydroxyethylcellulose, Hydroxypropylcellulose, Hydroxymethylcellulose, Polyvinylalkohol, Polyvinylpyrrolidon, Stärke und Gelatine;
das Vernetzungsmittel eines oder mehrere ist, ausgewählt aus Dihydroxyaluminiumaminoacetat, Magnesiumaluminometasilikat, Aluminiumhydroxid und synthetischem Hydrotalcit;
der Weichmacher Propylenglykol umfasst und ferner eines oder mehrere umfasst, ausgewählt aus Ethanol, Glycerin, 1,3-Butylenglykol, Polypropylenglykol, D-Sorbit und Polyethylenglykol 400; und
der Milchsäureester einer oder mehrere ist, ausgewählt aus Methyllactat, Ethyllactat, Propyllactat, Butyllactat, Pentyllactat, Hexyllactat, Heptyllactat, Octyllactat, Nonyllactat, Decyllactat, Undecyllactat, Lauryllactat, Tridecyllactat, Myristyllactat, Pentadecyllactat, Cetyllactat, Heptadecyllactat und Octadecyllactat;
und die pastöse Zubereitung keinen druckempfindlichen Klebstoff enthält.

2. Transdermale Formulierung nach Anspruch 1, wobei
das Verdickungsmittel eines oder mehrere ist, ausgewählt aus Carboxymethylcellulose-Natrium, Hydroxyethylcellulose, Hydroxypropylcellulose und Hydroxymethylcellulose;
das Vernetzungsmittel eines oder mehrere ist, ausgewählt aus Dihydroxyaluminiumaminoacetat, Magnesiumaluminometasilikat und synthetischem Hydrotalcit; und
der Milchsäureester einer oder mehrere ist, ausgewählt aus Lauryllactat, Myristyllactat und Cetyllactat.

3. Transdermale Formulierung nach einem der Ansprüche 1 bis 2, wobei der Weichmacher Propylenglykol umfasst und ferner eines oder mehrere umfasst, ausgewählt aus Ethanol, Glycerin und D-Sorbitol.

4. Transdermale Formulierung nach einem der Ansprüche 1 bis 3, wobei der Weichmacher eine Kombination aus Propylenglykol und Ethanol ist.

5. Transdermale Formulierung nach einem der Ansprüche 1 bis 4, wobei
die Polyacrylsäure oder ein Salz davon Polyacrylsäure ist;
das Verdickungsmittel Hydroxyethylcellulose ist;
das Vernetzungsmittel Magnesiumaluminometasilikat ist;
der Weichmacher eine Kombination aus Propylenglykol und Ethanol ist; und
der Milchsäureester einer oder mehrere ist, ausgewählt aus Lauryllactat, Myristyllactat und Cetyllactat.

6. Transdermale Formulierung nach einem der Ansprüche 1 bis 5, wobei die Menge des Testosterons 0,01 bis 10 Gew.-%, die Menge der Polyacrylsäure oder des Salzes davon 1 bis 20 Gew.-%, die Menge des Verdickungsmittels 0,5 bis 10 Gew.-%, die Menge des Vernetzungsmittels 0,02 bis 5 Gew.-%, die Menge des Weichmachers 45 bis 90 Gew.-% und die Menge des Milchsäureesters 0,1 bis 10 Gew.-%, bezogen auf das Gewicht der pastösen Zubereitung, beträgt.

7. Transdermale Formulierung nach einem der Ansprüche 1 bis 6, wobei die Menge des Testosterons 0,2 bis 4 Gew.-%, die Menge der Polyacrylsäure oder des Salzes davon 3 bis 15 Gew.-%, die Menge des Verdickungsmittels 1 bis 5 Gew.-%, die Menge des Vernetzungsmittels 0,05 bis 4 Gew.-%, die Menge des Weichmachers 67 bis 90 Gew.-% und die Menge des Milchsäureesters 0,5 bis 5 Gew.-%, bezogen auf das Gewicht der pastösen Zubereitung, beträgt.

8. Transdermale Formulierung nach einem der Ansprüche 1 bis 7 zur Verwendung bei der Vorbeugung oder Behandlung einer oder mehrerer Krankheit(en) oder eines oder mehrerer Symptome, ausgewählt aus Hypogonadismus, männlicher Menopause, metabolischem Syndrom, Diabetes, Insulinresistenz, Fettleibigkeit, Arteriosklerose, Osteoporose, Muskelschwäche, kognitivem Verfall, Gedächtnisstörungen und Depression.

## Revendications

1. Formulation transdermique comprenant de la testostérone ; une acide polyacrylique ou un de ses sels ; un épaississant ; un agent de réticulation ; un plastifiant comprenant du propylène glycol ; et un ester d'acide lactique dans une préparation pâteuse, dans laquelle
l'acide polyacrylique ou un de ses sels est choisi parmi l'acide polyacrylique, le polyacrylate de sodium et l'acide polyacrylique partiellement neutralisé ;
l'épaississant est un ou plusieurs agents choisis parmi la carboxyméthylcellulose sodique, l'hydroxyéthylcellulose, l'hydroxypropylcellulose, l'hydroxyméthylcellulose, l'alcool polyvinylique, la polyvinylpyrrolidone, l'amidon et la gélatine ;
l'agent de réticulation est un ou plusieurs agents choisis parmi l'aminoacétate de dihydroxyaluminium, l'aluminométasilicate de magnésium, l'hydroxyde d'aluminium et l'hydrotalcite synthétique ;
le plastifiant comprend du propylène glycol et comprend un ou plusieurs agents choisis parmi l'éthanol, la glycérine, le 1,3-butylène glycol, le polypropylène glycol, le D-sorbitol et le polyéthylène glycol 400 ; et l'ester d'acide lactique est un ou plusieurs composés choisis parmi le lactate de méthyle, le lactate d'éthyle, le lactate de propyle, le lactate de butyle, le lactate de pentyle, le lactate d'hexyle, le lactate d'heptyle, le lactate d'octyle, le lactate de nonyle, le lactate de décyle, lactate d'undécyle, lactate de lauryle, lactate de tridécyle, lactate de myristyle, lactate de pentadécyle, lactate de cétyle, lactate d'heptadécyle et lactate d'octadécyle ;
et la préparation pâteuse ne comprend pas d'adhésif sensible à la pression.

2. Formulation transdermique selon la revendication 1, dans laquelle
l'épaississant est un ou plusieurs agents choisis parmi la carboxyméthylcellulose sodique, l'hydroxyéthylcellulose, l'hydroxypropylcellulose et l'hydroxyméthylcellulose ;
l'agent de réticulation est un ou plusieurs agents choisis parmi l'aminoacétate de dihydroxyaluminium, l'aluminométasilicate de magnésium et l'hydrotalcite synthétique ; et
l'ester d'acide lactique est un ou plusieurs composés choisis parmi des lactates de lauryle, de myristyle et de cétyle.

3. Formulation transdermique selon l'une des revendications 1 à 2, dans laquelle le plastifiant comprend du propylène glycol et comprend en outre un ou plusieurs composés choisis parmi l'éthanol, la glycérine et le D-sorbitol.

4. Formulation transdermique selon l'une des revendications 1 à 3, dans laquelle le plastifiant est une combinaison de propylène glycol et d'éthanol.

5. Formulation transdermique selon l'une des revendications 1 à 4, dans laquelle
l'acide polyacrylique ou un de ses sels est de l'acide polyacrylique ;
l'épaississant est l'hydroxyéthylcellulose ;
l'agent de réticulation est l'aluminométasilicate de magnésium ;
le plastifiant est une combinaison de propylène glycol et d'éthanol ; et
l'ester d'acide lactique est un ou plusieurs des lactates de lauryle, de myristyle et de cétyle.

6. Formulation transdermique selon l'une des revendications 1 à 5, dans laquelle la quantité de testostérone est de 0,01 à 10% en poids, la quantité d'acide polyacrylique ou d'un de ses sels est de 1 à 20% en poids, la quantité d'épaississant est de 0,5 à 10% en poids, la quantité d'agent de réticulation est de 0,02 à 5% en poids, la quantité de plastifiant est de 45 à 90% en poids, et la quantité d'ester d'acide lactique est de 0,1 à 10% en poids, par rapport au poids de la préparation pâteuse.

7. Formulation transdermique selon l'une des revendications 1 à 6, dans laquelle la quantité de testostérone est de 0,2 à 4% en poids, la quantité d'acide polyacrylique ou d'un de ses sels est de 3 à 15% en poids, la quantité d'épaississant est de 1 à 5% en poids, la quantité d'agent de réticulation est de 0,05 à 4% en poids, la quantité de plastifiant est de 67 à 90% en poids, et la quantité d'ester d'acide lactique est de 0,5 à 5% en poids, par rapport au poids de la préparation pâteuse.

8. Formulation transdermique selon l'une des revendications 1 à 7, pour utilisation dans la prévention ou le traitement d'une ou plusieurs maladie(s) ou symptôme(s) choisi(s) parmi l'hypogonadisme, la ménopause masculine, le syndrome métabolique, le diabète, la résistance à l'insuline, l'obésité, l'artériosclérose, l'ostéoporose, la faiblesse musculaire, le déclin cognitif, les troubles de la mémoire, et la dépression.
